(19) Europäisches Patentamt

European Patent Office

Office européen des brevets

(11) EP 1 562 129 A1

(12) EUROPEAN PATENT APPLICATION

(43) Date of publication:
10.08.2005 Bulletin 2005/32

(51) Int Cl.⁷: G06F 17/50, C07K 1/00, G06F 19/00

(21) Application number: 04255094.7

(22) Date of filing: 25.08.2004

(84) Designated Contracting States:
AT BE BG CH CY CZ DE DK EE ES FI FR GB GR HU IE IT LI LU MC NL PL PT RO SE SI SK TR
Designated Extension States:
AL HR LT LV MK

(30) Priority: 25.12.2003 JP 2003429177

(71) Applicant: FUJITSU LIMITED
Kawasaki-shi, Kanagawa 211-8588 (JP)

(72) Inventors:
• Tomonaga, Atsushi, Fujitsu Limited
Kawasaki-shi, Kanagawa 211-8588 (JP)

• Shiobara, Noriyuki, Fujitsu Limited
Kawasaki-shi, Kanagawa 211-8588 (JP)
• Sugiyama, Hajime, Fujitsu Limited
Kawasaki-shi, Kanagawa 211-8588 (JP)

(74) Representative: Stebbing, Timothy Charles et al
Haseltine Lake,
Imperial House,
15-19 Kingsway
London WC2B 6UD (GB)

(54) **Method and apparatus for generating biologically-active-substance candidate structure**

(57) To generate a candidate compound, compound fragments (112 to 115) in a fragment library (101) are input into an active site (102) to perform initial packing. Using a field of force in which atoms repel each other when they are close energetically and attract each other when they are apart, so-called shaking is performed by an MD calculation, thereby stabilizing the compound fragments (112 to 115) packed into the active site (102). Further, at obtained stable locations of the compound fragments, monoatoms are packed and fragments are bonded together. As a result, a candidate ligand structure (103) is output.

FIG.1

Printed by Jouve, 75001 PARIS (FR)

## Description

This application is based upon and claims the benefit of priority from the prior Japanese Patent Application No.2003-429177, filed on December 25, 2003.

The present invention relates to a method and an apparatus for generating a biologically-active-substance candidate structure and a computer product for generating a structure of a novel biologically-active-substance candidate based on a protein form, such as a medical, pharmaceutical, or agricultural product, as well as to a method and system for manufacturing a biologically-active substance based on such a generated structure.

Conventional methods of generating a candidate compound are roughly classified as follows. A first method is to design a scaffold based on common element extraction by superposing known drug molecules. A second method is to obtain bonding energies of large quantities of compounds in a library with a protein, respectively, by a molecular dynamics calculation or the like, and to screen candidates that are possibly bonded with the protein (a screening method by a docking simulation). A third method is to obtain a scaffold by selecting some partial structures that may match to a protein-side amino acid, and bonding the selected partial structures using a spacer (see, for example, Japanese Patent Application Laid-Open No. H7-133233). A fourth method is to pack pseudo atoms into an active site, and find a possible scaffold structure from a most closely packed site (see, for example, Japanese Patent Application Laid-Open No. 2000-178209 Publication).

However, the conventional methods have the following problems. Regarding the first method, the designed structure is limited to being a less-novel structure, i.e. to being similar to a well-known structure. In the second method, accurate obtaining of the bonding energies at a high speed is not always established, so that proper candidates cannot be selected. With the third method, despite much candidate structure generation software adopting this method, an important element for candidate structures of "complementarity with a form of the protein active site" tends to be disadvantageously overlooked. The fourth method is the most highly evaluated method in terms of not missing possible candidates. However, the scaffold structure obtained by bonding packed pseudo atomic spheres is estimated to contain stereochemically large distortion, and candidate structures which are eventually difficult to synthesize may possibly be included in the candidates.

It is therefore desirable to solve at least the above problems in the conventional technology.

An apparatus for generating a biologically-active-substance candidate structure according to one aspect of the present invention includes a first unit that selects fragments of an arbitrary compound, a second unit that stably locates the fragments selected, and a third unit that inputs pseudo monoatoms into the active site in which the arbitrary fragments are stably located, and constructs a molecular scaffold by bonding the fragments together, bonding the fragments with the monoatoms, and bonding the monoatoms together.

A method of generating a biologically-active-substance candidate structure according to another aspect of the present invention includes selecting fragments of an arbitrary compound, stably locating the fragments selected, and inputting pseudo monoatoms into the active site in which the arbitrary fragments are stably located and constructing a molecular scaffold by bonding the fragments together, bonding the fragments with the monoatoms, and bonding the monoatoms together.

A computer readable recording medium according to another aspect of the present invention stores a computer program that realizes the method according to the above aspect on a computer.

The results of the above apparatus and method may be stored for use in synthesizing novel biologically-active substances by synthesizing means, which may be conventional per se and thus will not be described.

Other features and advantages of the present invention are specifically set forth in or will become apparent from the following detailed description of the invention when read in conjunction with the accompanying drawings, in which:

Fig. 1 is a schematic for explaining an outline of a biologically-active-substance candidate structure generation method according to an embodiment of the present invention;
Fig. 2 is a flowchart of a process procedure for the biologically-active-substance candidate structure generation method according to the embodiment;
Fig. 3 is a schematic for illustrating an example of a hardware configuration of a biologically-active-substance candidate structure generating apparatus according to the embodiment;
Fig. 4 is a schematic for illustrating a functional configuration of the biologically-active-substance candidate structure generating apparatus according to the embodiment;
Fig. 5 is a flowchart of a process procedure for a fragment selecting process according to the embodiment;
Fig. 6 is a flowchart of a process procedure for an MD calculation process according to the embodiment;
Fig. 7 is a flowchart of a process procedure for monoatoms locating, binding probability estimation, and molecular scaffold construction processing according to the embodiment;
Fig. 8 is a table of conditions for determining pseudo atom locations according to the embodiment;
Fig. 9 is a schematic for illustrating a content of the condition (5) in the table shown in Fig. 8;
Fig. 10 is a flowchart of a process procedure for the heteroatom substituent candidate selection, hetero atom substitution estimation, and determination processing according to the embodiment;

Fig. 11A is a schematic for illustrating a state of a measured location on the x-y plane;

Fig. 11B is a schematic for illustrating a state of a measured location on the x-z plane;

Fig. 12A is a schematic for illustrating a state of a packing result on the x-y plane;

Fig. 12B is a schematic for illustrating a state of a packing result on the x-z plane;

Fig. 13 is a schematic for explaining an example of a known inhibitor structure;

Fig. 14 is a schematic for explaining a first example of a candidate ligand structure;

Fig. 15 is a schematic for explaining a second example of the candidate ligand structure;

Fig. 16 is a schematic for explaining a third example of the candidate ligand structure;

Fig. 17 is a schematic of a three-dimensional structure of the known inhibitor shown in Fig. 13; and

Fig. 18 is a schematic of a three-dimensional structure of the candidate ligand shown in Fig. 14.

Exemplary embodiments of a method and an apparatus for generating a biologically-active-substance candidate structure, and a computer product according to the present invention will be explained below in detail with reference to the accompanying drawings.

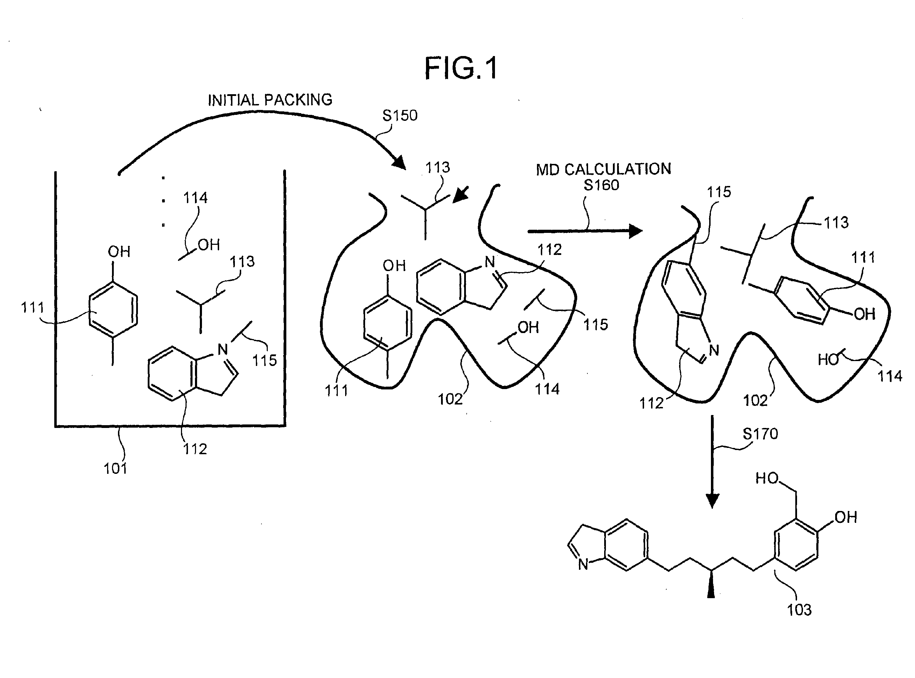

Fig. 1 is a schematic for explaining an outline of a biologically-active-substance candidate structure generation method according to an embodiment of the present invention.

Several partial structures, which can be synthesized and are often applied as drugs, are first selected, and located in an active site of a protein. Thereafter, small partial structures or monoatoms are located therein so as to provide an overall location which is energetically stable. A molecular dynamics calculation is used for location searches. As the molecular dynamics calculation, a molecular dynamics (MD) method, a Monte Carlo (MC) method, or the like can be used. It is considered that the MD method is the most efficient method.

While searching for a location at which the partial structures satisfactorily interact with the protein active site and at which the partial structures can be satisfactorily bonded together, the respective partial structures and monoatoms are coupled to thereby obtain a candidate structure. Thus, a candidate structure which has both novelty and synchronization probability can be generated.

Specifically, as shown in Fig. 1, a reference sign 101 denotes a compound fragment library, 102 denotes the active site, 103 denotes a candidate ligand structure of a candidate ligand compound (e.g., an inhibitor), and 111 to 115 denote compound fragments, respectively.

The compound fragments 111 to 115 in the fragment library 101 are input into the active site 102, thereby performing initial packing (at S150). Thereafter, using a field of force in which atoms repel each other when they are energetically close and attract each other when they are away (apart), the active site 102 is subjected to so-called shaking to stabilize the compound fragments 111 to 115 packed into the active site 102 (at S160). Further, at the obtained stable location of the compound fragments 111 to 115, monoatoms are packed into the active site 102, thereby bonding the fragments together (at a step S170). As a result, the candidate ligand structure 103 is output.

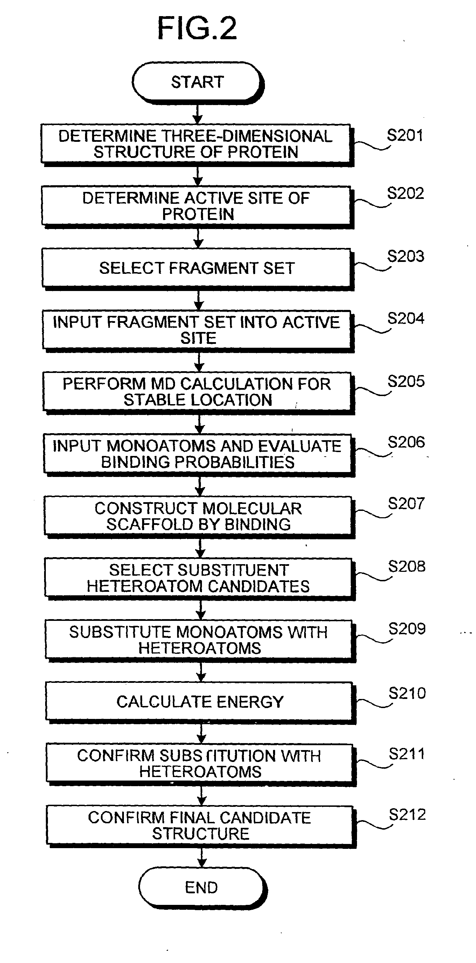

Fig. 2 is a flowchart of a process procedure for the biologically-active-substance candidate structure generation method according to the embodiment. A three-dimensional structure of the protein is determined first (at a step S201), and the target active site 102 of the protein is determined (at a step S202).

A fragment set of the compound fragments to be packed (input) are selected from the fragment library 101 (at a step S203). The selected fragment set is input into the active site 102 (at a step S204), and an MD calculation is performed so as to stably locate the input fragment set in the active site 102. In order to stably locate the input fragment set, it is necessary to set "positions" and "orientations" of the fragments relative to the active site.

Thereafter, while the fragments are stably located as obtained, pseudo monoatoms are packed (input) into the active site, and their respective binding probabilities are evaluated (at a step S206). A molecular scaffold by binding is constructed (at a step S207), heteroatom substituent candidates are selected (at a step S208), and the pseudo monoatoms are substituted with the selected heteroatoms (at a step S209). At this time, an energy calculation is performed while the pseudo monoatoms are substituted with the selected heteroatoms (at a step S210), and it is confirmed that the pseudo monoatoms are substituted with the selected heteroatoms (at a step S211). Finally, a final candidate structure is confirmed (at a step S212), and the series of processings are finished.

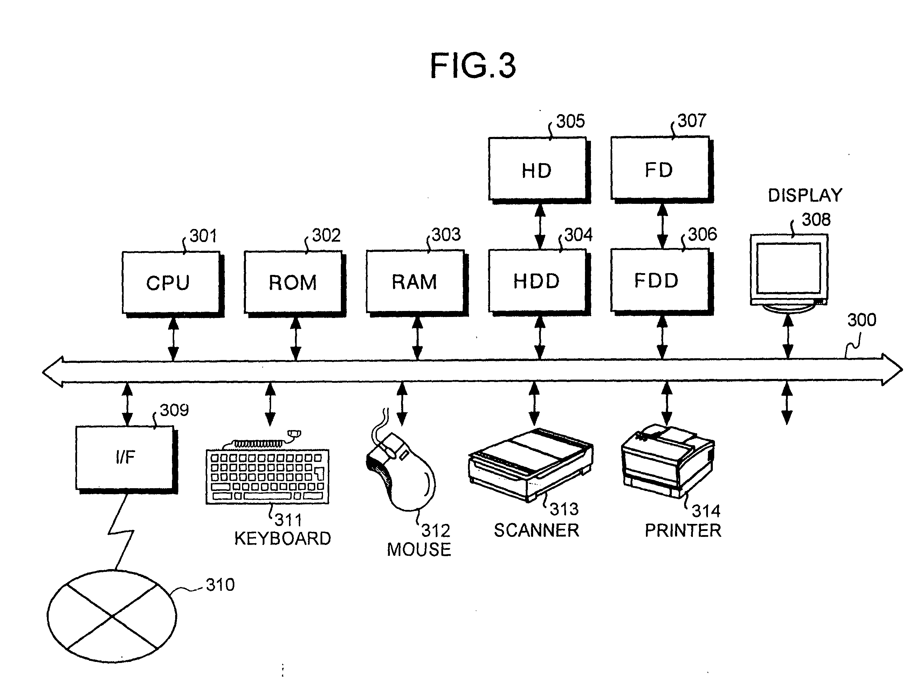

Fig. 3 is a schematic for illustrating an example of a hardware configuration of a biologically-active-substance candidate structure generating apparatus according to the embodiment.

A biologically-active-substance candidate structure generating apparatus includes a central processing unit (CPU) 301, a read only memory (ROM) 302, a random access memory (RAM) 303, a hard disk drive (HDD) 304, a hard disk (HD) 305, a flexible disk drive (FDD) 306, a flexible disk (FD) 307 that is one example of a detachable recording medium, a display 308, an interface (I/F) 309, a keyboard 311, a mouse 312, a scanner 313, and a printer 314. The respective constituent elements of the apparatus are connected to one another by a bus 300.

The CPU 301 controls the entirety of the biologically-active-substance candidate structure generating apparatus. The ROM 302 stores programs such as a boot program. The RAM 303 is used as a work area for the CPU 301. The HDD 304 controls read and write of data from and to the HDD 305 under control of the CPU 301. The HD 305 stores the written data under control of the HDD 304.

The FDD 306 controls read and write of data from and to the FD 307 under control of the CPU 301. The FD 307 stores the written data under control of the FDD 306, and reads the data recorded in the FD 307 to an information processing apparatus. As the detachable recording medium, a CD-ROM (CD-R or CD-RW), a magneto-optical (MO) disk, a digital versatile disk (DVD), a memory card, or the like may be employed instead of the FD 307. The display 308 displays data such as a document, an image, and functional information as well as cursors, icons, or tool boxes. The display 308 is, for example, a cathode ray tube (CRT), a thin film transistor (TFT) liquid crystal display (LCD), or a plasma display panel (PDP).

The I/F 309 is connected to a network 310 such as a local area network (LAN) or the Internet through a communication line, and is connected to the other server or the information processing apparatus including a database and the like through the network 310. The I/F 309 interfaces the network 310 with the constituent elements of the biologically-active-substance candidate structure generating apparatus, and controls input and output of data from and to the other server or an information terminal apparatus. The I/F 309 is, for example, a modem or a LAN adapter.

The keyboard 311 includes keys used to input characters, numbers, various instructions, and the like, and inputs data. The keyboard 311 may be replaced by a touch-panel input pad or a numeric keypad. The mouse 312 performs a cursor movement, a range selection, a window movement, a size change, and the like. The mouse 312 may be replaced by a trackball, a joystick, or the like as long as it functions equally to the mouse 312 as a pointing device.

The scanner 313 optically reads an image such as a driver image and fetches image data into the information processing apparatus. The scanner 313 also has an optical character recognition (OCR) function, and can read printed information and convert the read information into data by the OCR function. The printer 314 prints image data and document data. The printer 314 is, for example, a laser printer or an inkjet printer.

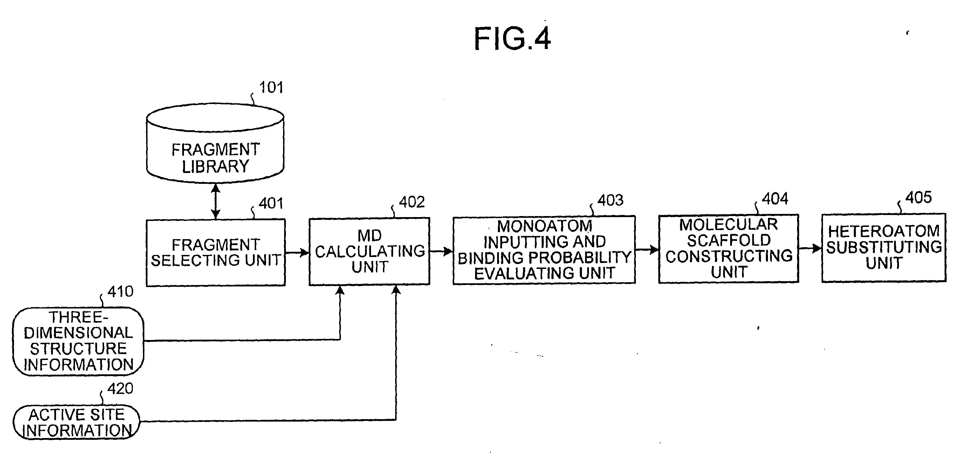

Fig. 4 is a schematic for illustrating a functional configuration of the biologically-active-substance candidate structure generating apparatus according to the embodiment.

The biologically-active-substance candidate structure generating apparatus includes a fragment selecting unit 401, an MD calculating unit 402, a monoatom inputting and binding probability evaluating unit 403, a molecular scaffold constructing unit 404, and a heteroatom substituting unit 405.

The fragment selecting unit 401 selects arbitrary compound fragments from the fragment library 101. The fragment selecting unit 401 may select so-called large fragments each having a predetermined number of atoms or more and a predetermined volume or more, and then select so-called small fragments each having a number of atoms less than the predetermined number of atoms and a volume less than the predetermined volume.

The MD calculating unit 402 conducts a location search using the molecular dynamics calculation (e. g., MD calculation) based on protein three-dimensional information 410 and active site information 420 so as to stably locate the fragments selected by the fragment selecting unit 401 in the active site of the protein. As a result, stable locations of the fragments can be obtained.

The monoatom inputting and binding probability evaluating unit 403 inputs pseudo monoatoms (including small partial structures) into the active site in which the arbitrary fragments are stably located, and evaluates (determines) binding probabilities of bonding between the fragments, bonding between the fragments and the monoatoms, and bonding between the monoatoms. Specifically, the monoatom inputting and binding probability evaluating unit 403 determines the binding probabilities based on whether at least, for example, atomic distances and bond angles are within their allowable ranges.

The molecular scaffold constructing unit 404 constructs a molecular scaffold by bonding the fragments together, bonding the fragments and the monoatoms, and bonding the monoatoms together based on an evaluation result of the monoatom inputting and binding probability evaluating unit 403.

The heteroatom substituting unit 405 substitutes the pseudo monoatoms, for which the molecular scaffold is constructed by the molecular scaffold constructing unit 404, with heteroatoms. Specifically, based on, for example, a fluctuation in energy of an electrostatic interaction, the heteroatom substituting unit 405 determines whether substitution with the heteroatoms is effective, and substitutes the pseudo atoms with the heteroatoms based on the determination result.

The functions of the fragment selecting unit 401, the MD calculating unit 402, the monoatom inputting and binding probability evaluating unit 403, the molecular scaffold constructing unit 404, and the heteroatom substituting unit 405 are realized specifically when, for example, the CPU 301 executes the program recorded in the ROM 302, the RAM 303, the HD 305 or FD 307 shown in Fig. 3.

The fragment library 101 is composed by, for example, a first library for real atoms (carbon atoms and heteroatoms) and a second library for pseudo monoatoms obtained by converting all the real atoms.

The first library for the real atoms (carbon atoms and heteroatoms) is a database which stores partial structures that appear in biologically-active-substances such as existing medical, agricultural, and pharmaceutical products with high frequency in the form of molecular structural formulae (three-dimensional coordinates). Further, since amino acids are often applied as constituent elements of medical and pharmaceutical products, side chains (of e.g. 20 types) of amino acids may be stored in the first library. A minimum unit is a C of an alanine side chain (one carbon atom).

In addition, the second library for the pseudo monoatoms converted from all the real atoms of the first library stores, for example, only patterns such as three-membered, four-membered, five-membered, six-membered, and seven-membered rings, condensed rings, linear scaffolds, and monoatoms. Specifically, the function of the fragment library 101 is realized by, for example, the HD 305 and the FD 307 shown in Fig. 3. Further, the fragment library 101 may be included in, for example, the other information processing apparatus connected to the biologically-active-substance candidate structure generating apparatus through the I/F 309 by the network 310 shown in Fig. 3.

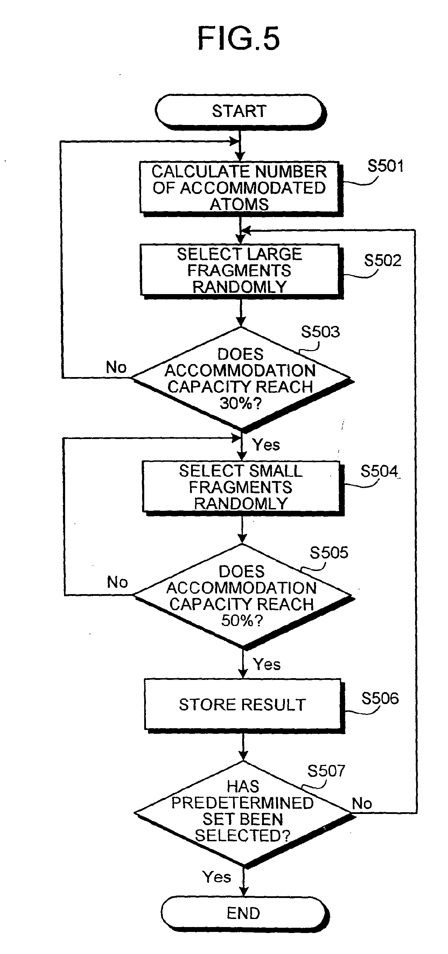

Fig. 5 is a flowchart of a process procedure for a fragment selecting process according to the embodiment. It is assumed that a rectangular parallelepiped which accommodates the active site 102 of a target protein is present, and the number of atoms accommodated in the rectangular parallelepiped is calculated (at a step S501).

Several large-sized fragments (large fragments), specifically, fragments each having a predetermined number of atoms or more or a predetermined volume or more are randomly selected (at a step S502). The large fragments are normally cyclic compounds or the like. It is determined whether a total number of atoms account for an accommodation capacity of, for example, about 30% of the number of atoms accommodated in the rectangular parallelepiped (at a step S503). The selection is continued until the accommodation capacity reaches about 30%. If the accommodation capacity reaches about 30% ("Yes" at the step S503), the small fragments are randomly selected (at a step S504) and the small fragments are added until the accommodation capacity reaches about 50%.

If the accommodation capacity reaches about 50% ("Yes" at the step S505), the result is stored (at a step 5506). It is then determined whether a preset predetermined number of fragment sets are selected (at a step S507). If the predetermined number of fragment sets are not selected yet ("No" at the step S507), the processing returns to the step S502. Thereafter, the steps S502 to S507 are repeatedly executed. If the predetermined number of fragment sets are selected ("Yes" at the step S507), the series of processings is finished.

It is noted that 30% and 50% explained above are examples, and that optimum values may be selected according to a state of the active site and the type of fragments to be input.

An initial packing quantity is set at, for example, 180 fragments/$nm^3$ when the rectangular parallelepiped is most closely packed in the form of diamond (at a density of 3.51 $g/cm^3$). However, the most closely packed rectangular parallelepiped may be too dense, so that the initial packing quantity can be set in a range of 30 fragments/$nm^3$ to 90 fragments/$nm^3$. In addition, if the protein is an HIV protease inhibitor (PDB: 1D4H), an active site space has a volume of about 0.8 $nm^3$ and the inhibitor has 44 atoms (except for hydrogen atoms). Therefore, the initial packing quantity is 55 fragments/$nm^3$. For most drugs (finally packed state), the initial packing quantity is set at about 55 fragments/$nm^3$. In this embodiment, the initial packing quantity is far smaller than 55 fragments/$nm^3$, i.e., about 20 fragments/$nm^3$ to 40 fragments/$nm^3$. In this state, monoatoms are packed into the rectangular parallelepiped.

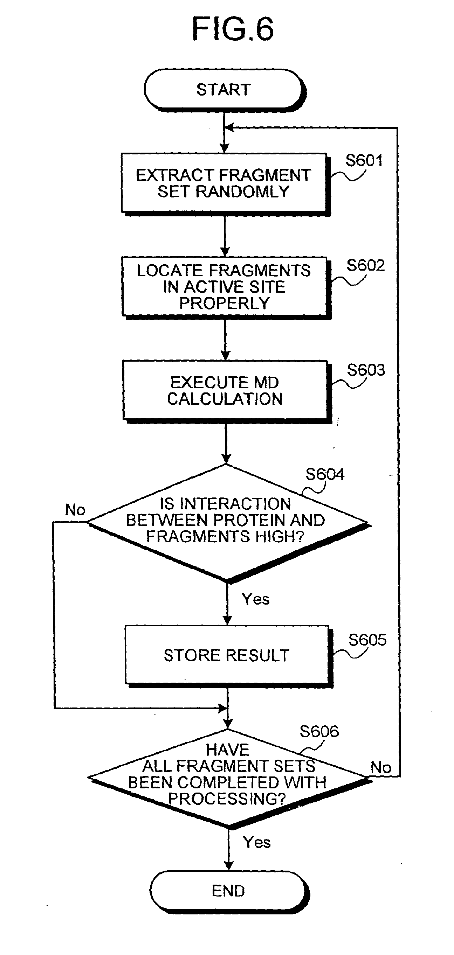

Fig. 6 is a flowchart of a process procedure for an MD calculation process according to the embodiment. One fragment set is randomly extracted from the selected predetermined number of fragment sets (at a step S601). The fragments are appropriately located in the active site (at a step S602).

An MD calculation based on an interaction between the protein and the compound and an interaction between the fragments is executed (at a step S603). The interaction between the protein and the fragment is evaluated and it is determined whether the interaction is higher than a predetermined index (at a step S604). If the interaction is higher than the predetermined index ("Yes" at the step S604), the result is stored (at a step S605). If the interaction is lower than the predetermined index ("No" at the step S604), no processing is performed.

As a result, the fragments which are located in an area relatively close to a lining wall of the protein remain. If a calculation time is short, then all fragments cannot be searched and the result may possibly depend on the initial location of the fragments (a relative position and a direction of each fragment) in the protein active site. The MD calculation may be performed for the fragments having different initial location patterns.

It is determined whether processing has been completed for all of the predetermined number of fragment sets (at a step S606). If all the predetermined number of fragments have not been processed ("No" at the step S606), the processing returns to the step S601. Thereafter, the steps S601 to S606 are repeatedly executed. At the step S606, if processing is completed for all the predetermined number of fragments ("Yes" at the step S606), the series of processings is finished.

In the present embodiment, after the predetermined number of fragment sets are selected, the MD calculation processing is performed. Alternatively, the MD calculation processing may be performed whenever fragments are selected.

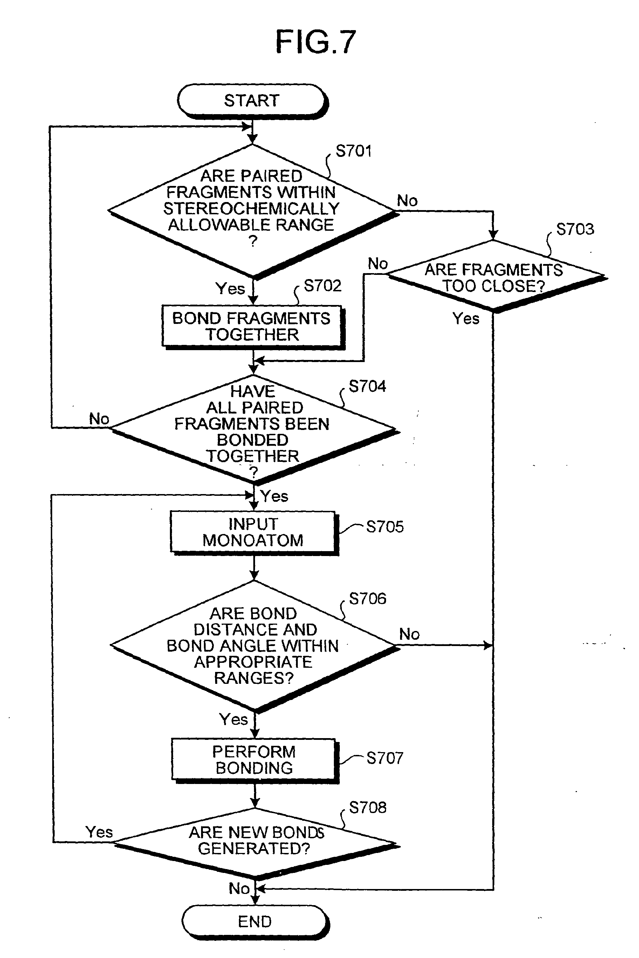

Fig. 7 is a flowchart of a process procedure for monoatoms locating, binding probability estimation, and molecular scaffold construction processing according to the embodiment. Two fragments for which the atomic distance and the bond angle are within stereochemically allowable ranges, respectively are searched (at a step S701).

If the atomic distance and the bond angle of the fragments are within their respective stereochemically allowable ranges ("Yes" at the step S701), the fragments are bonded together (at a step S702). If the atomic distance and the bond angle thereof are not within stereochemically allowable ranges ("No" at the step S701) and the fragments are too close ("Yes" at a step S703), the fragments are excluded from being candidates and the processing is finished. If the atomic distance and the bond angle thereof are not within stereochemically allowable ranges ("No" at the step S701) and the fragments are apart (distanced) from each other ("No" at a step S703), no processing is performed. It is determined whether all fragments have been bonded together (at a step S704). If all fragments have not been bonded yet ("No" at the step S704), the processing returns to the step S701.

If all fragments have been bonded together ("Yes" at the step S704), many gaps are present everywhere, with the fragments located only in the active site. Therefore, to fill the gaps, monoatoms are input (at a step S705) and a location at which binding can be expected is searched for. Specifically, one atom is input at an arbitrary location, and it is determined whether a new bonding distance and a new bond angle formed between the input atom and a certain atom of the fragment already located or a monoatom input after the one atom are within appropriate ranges (at a step S706).

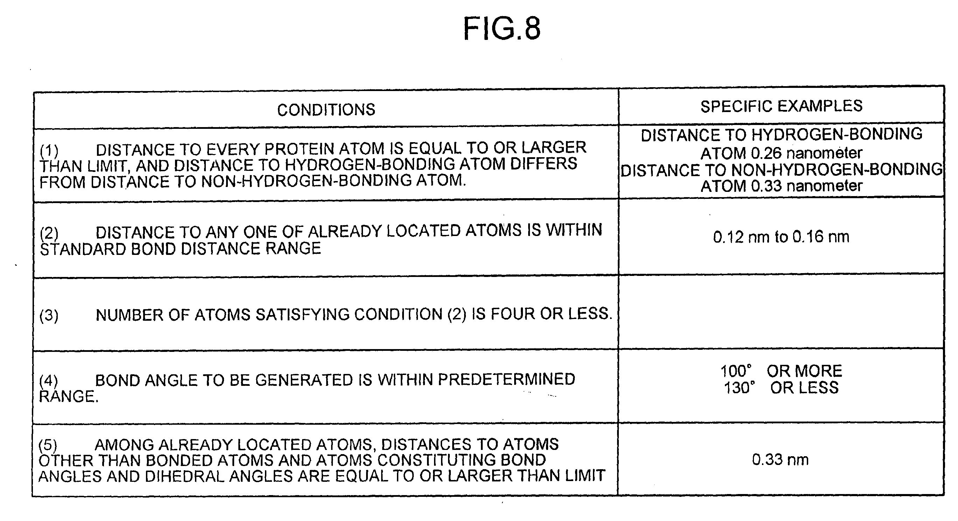

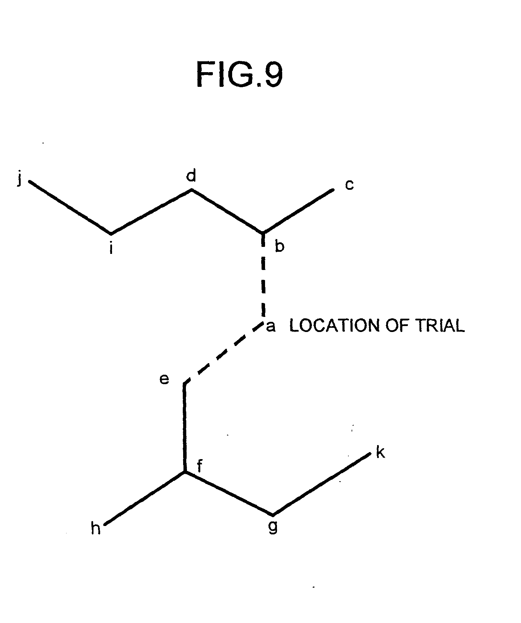

Whether the new bonding distance and the new bond angle are within their respective appropriate ranges is determined based on, for example, conditions for determining whether a pseudo atom can be located as shown in Fig. 8. Specifically, while the bonding distance is set at, for example, 0.12 nm to 0.16 nm and the bond angle is set at, for example, 100 degrees to 130 degrees, all atoms which can be bonded with the one atom are searched. For the bond angle, it is determined whether a bond angle formed between a newly formed bond and an originally present bond ahead of the new bond is within the allowable range. Fig. 9 is a schematic for illustrating a content of the condition (5) in the table shown in Fig. 8. The condition (5) in the table indicates that if a tried location position is a, then Rab and Rae are within the allowable range, and θbae, θabc, θabd, and θaef are within the allowable range, and that Daj, Dak, and the like are equal to or larger than a limited distance (within the allowable range). Symbols Rab, Rae, Daj, and Dak denote distances between a and b, a and e, a and j, and a and k, respectively, and symbols θbae, θabc, θabd, and θaef denote angles formed between ba and ea, ab and cb, ab and db, and ae and fe, respectively.

If the bond angles and the bonding distances are within their respective appropriate ranges ("Yes" at the step S706), the one atom is coupled with all the other atoms (at a step S707). If they are not within the respective appropriate ranges ("No" at the step S706), the processing is finished. Further, a monoatom which may possibly be bonded is bonded with a certain atom of a fragment or another monoatom. If a plurality of candidate atoms bonded with the monoatom for which the bonding distances and the bond angles are within the allowable ranges are present, all of the candidate atoms are bonded with the one monoatom. For the one atom, bonding distances and bond angles are similarly calculated and binding probabilities are calculated for an atom (a part of the fragment or another monoatom) ahead of the one atom. If it is determined that candidates can be bonded with the atom, they are bonded together.

If the processings are repeatedly executed ("Yes" at a step S708), the gaps become smaller and smaller. At the same time, fragments and monoatoms are gradually bonded, so that the structure is larger. If no new bond is generated anywhere after repeating the processings at the steps S705 to S707 ("No" at the step S708), the series of processings are finished.

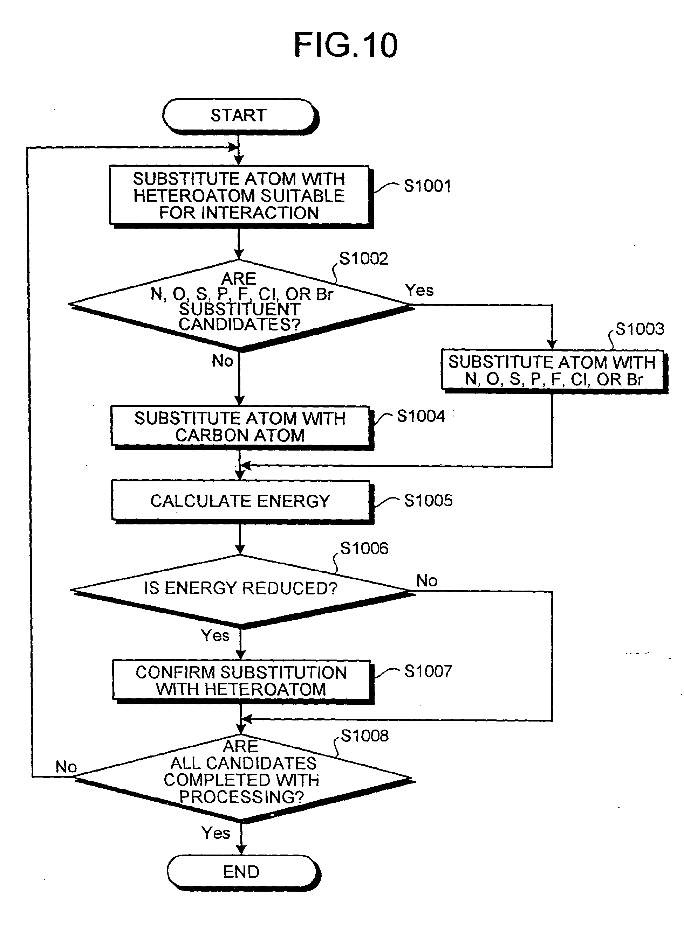

Fig. 10 is a flowchart of a process procedure for the heteroatom substituent candidate selection, hetero atom substitution estimation, and determination processing according to the embodiment.

A correlation between an atom and an amino acid of the protein is observed and the atom is substituted with a heteroatom suitable for an interaction with the amino acid (at a step S1001). A negatively charged atom is located for a positively charged atom or vice versa, or an atom which serves as a hydrogen-bond acceptor is located for an atom which serves as a hydrogen-bond donor or vice versa. If N, O, S, P, F, Cl, Br, or the like is a substituent candidate ("Yes" at a step S1002), the atom is substituted with the substituent candidate (at a step S1003). Otherwise ("No" at the step S1002), the atom is substituted with a carbon atom (at a step S1004).

For heteroatom substitution, it is normally considered that many candidates are present. Attention is paid to one of the candidates, and energy of an electrostatic interaction is calculated so as to determine whether substitution of the atom with a certain heteroatom is effective (at a step S1005). The effectiveness is evaluated based on a fluctuation in the calculated energy. If the substitution is effective, that is, the energy is reduced ("Yes" at the step S1006), the substitution is adopted and it is confirmed that the atom is substituted with the heteroatom (at a step S1007). If it is not effective ("No" at the step S1006), the substitution is not adopted and the processing moves to a step S1008. Thereafter, similar operation is continued for the other location ("No" at a step S1008). If processing is completed for all the candidates ("Yes" at the step S1008), the series of processings is finished.

Since a dihedral angle is not considered so far, the candidates which have abnormal dihedral angles are excluded. In addition, in order to evaluate overall docking with the protein, free bonding energies are calculated and checked. Further, by making final confirmation based on an index of Drug-likeness such as the Lipinski rule, atoms which cannot be expected to serve as candidates are excluded. As a result, the final candidate structure can be confirmed and stored.

As explained so far, with the method and the apparatus for generating biologically-active-substance candidate structure and the computer product according to the embodiment, fragments of an arbitrary compound (e.g. a known drug) are selected, the selected fragments are stably located in the active site of the protein, pseudo monoatoms are input into the active site in which the arbitrary fragments are stably located, the molecular scaffold based on bonding between the fragments, bonding between the fragments and the monoatoms, and bonding between the monoatoms is constructed, and the pseudo monoatoms, for which the molecular scaffold is constructed, are substituted with heteroatoms. Therefore, a candidate ligand compound (e.g., an inhibitor) to be bonded with a protein (e.g., enzyme) having a known three-dimensional structure can be designed de novo. Further, according to the embodiment, in a process of searching candidate compounds which control (inhibit or promote) functions of the known protein, a hit ratio can be improved, a search time can be shortened, and a cost reduction can be realized.

The biologically-active-substance candidate structure generation method explained in the embodiment can be realized by allowing a computer such as a personal computer or a workstation to execute a program prepared in advance. The program is recorded in a computer readable recording medium such as a hard disk, a flexible disk, a CD-ROM, an MO disk, or a DVD, and executed by reading the program. Alternatively, this program may be a transmission medium which can be distributed through a network such as the Internet.

An example of the embodiment is as follows.

1. Materials

Protein: HIV protease (PDB number: 1D4H)
Drug: Inhibitor (Bea435, C36H38N207)

A ligand in an X-ray co-crystal structure is divided into fragments and it is examined whether a similar location of the fragments can be reproduced by the method according to the embodiment.

2. Set potential

Fragments: Partial structure of a rigid body (zero internal flexibility)
Atomic species: Pseudo atoms (mass of 20.179)
Potential: A potential with which only van der Waals force acts between the lining wall of the protein and the fragment and between the fragments is set (the following Leonard-Jones potential is used).
Leonard-Jones Potential

$$E_{VDW} = \varepsilon_{ij} \left[ (\sigma_{ij}/r)^{12} - 2 (\sigma_{ij}/r)^{6} \right] \qquad (1)$$

where $E_{VDW}$: van der Waals energy

r: Atomic distance
$\varepsilon_{VV}$: Pseudo atom, parameter between pseudo atoms
$\sigma_{VV}$: Pseudo atom, parameter between pseudo atoms
$\varepsilon_{VP}$: Pseudo atom, parameter between protein atoms
$\sigma_{VP}$: Pseudo atom, parameter between protein atoms

3. Molecular dynamics method

Software: TINKER
Calculation TimeStep: 1 fs
Calculation time: 20 ps
Temperature: 298K

Initial location: four cyclic structures of the 1D4H inhibitor are selected with positions and orientation set randomly.

Other conditions:

Step1: MM under the following conditions.
$\varepsilon_{VV}$=0.755, $\sigma_{VV}$=0.01, $\varepsilon_{VP}$=3.45, and $\sigma_{VP}$=0.02
Step2: MD for 20 ps under the following conditions.
$\varepsilon_{VV}$=0.755, $\sigma_{VV}$=0.01, $\varepsilon_{VP}$=3.45, $\sigma_{VP}$=2.0, and temperature of 298K
Step3: MD for 20 ps under the following conditions.
$\varepsilon_{VV}$=0.755, $\sigma_{VV}$=0.01, $\varepsilon_{VP}$=3.45, $\sigma_{VP}$=2.06, and temperature of 298K

4. Result and evaluation

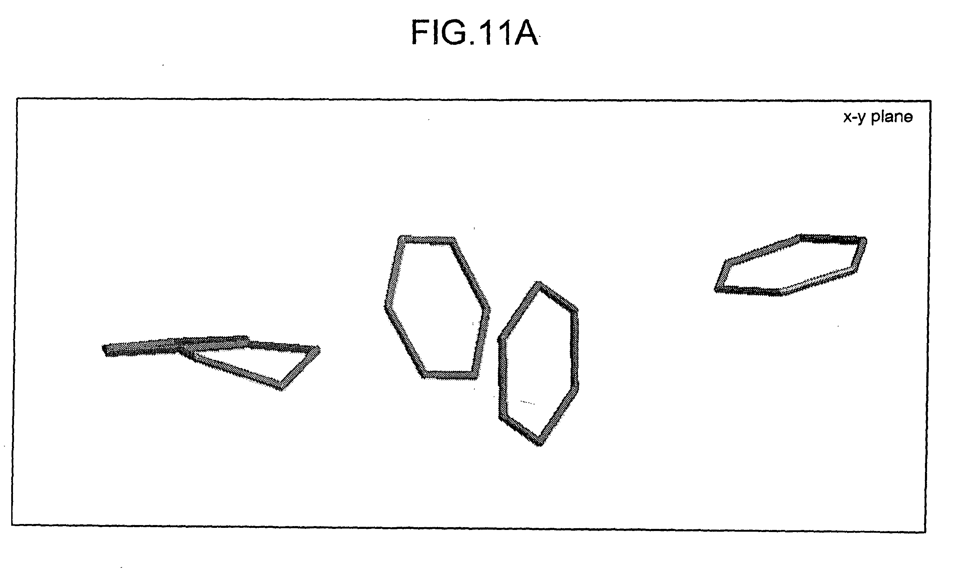

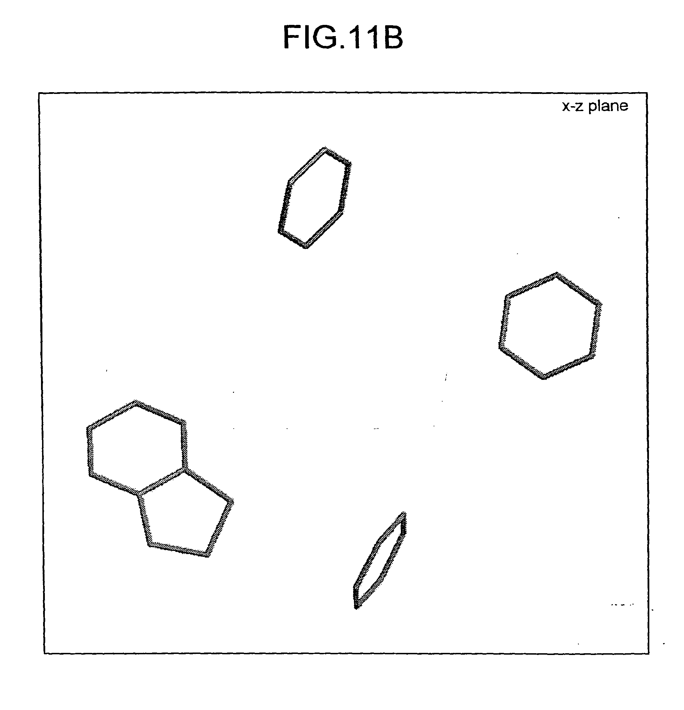

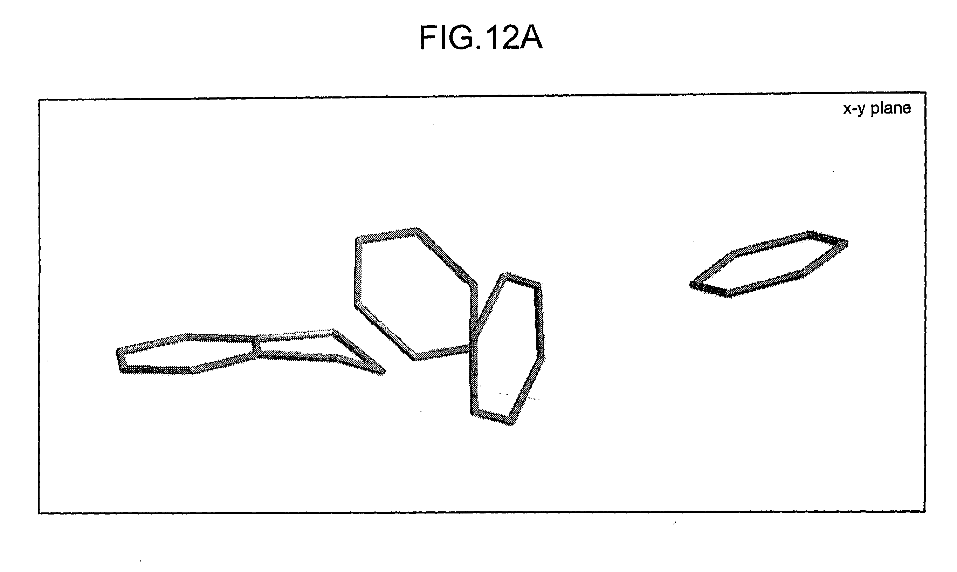

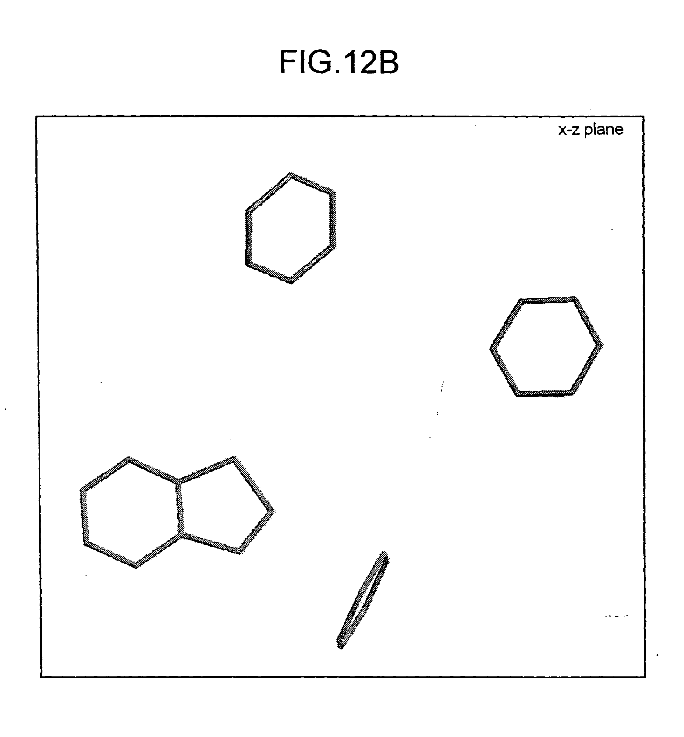

The following result is obtained when the initial location is changed and the processing is performed. Fig. 11A is a schematic for illustrating a state of a measured location on the x-y plane; and Fig. 11 B is a schematic for illustrating a state of a measured location on the x-z plane. Fig. 12A is a schematic for illustrating a state of a packing result on the x-y plane; and Fig. 12B is a schematic for illustrating a state of a packing result on the x-z plane.

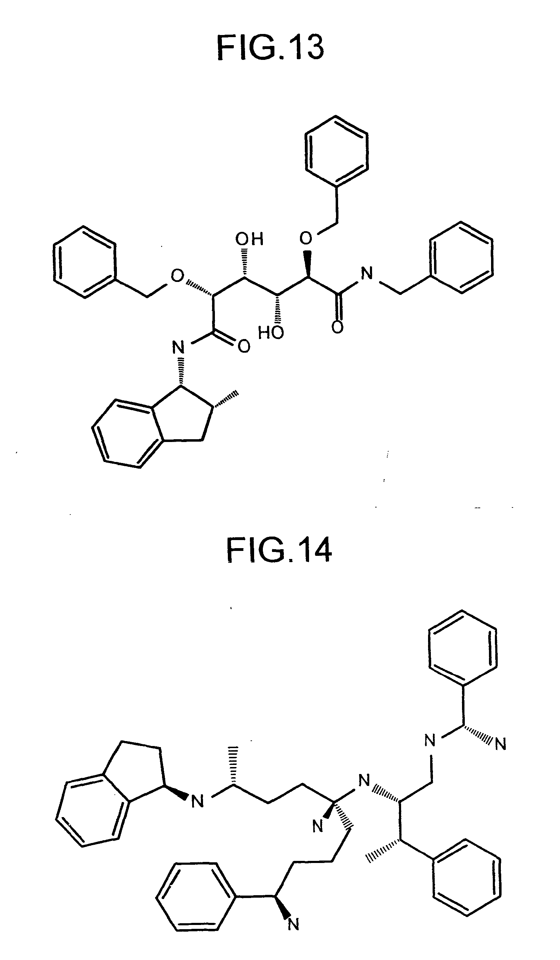

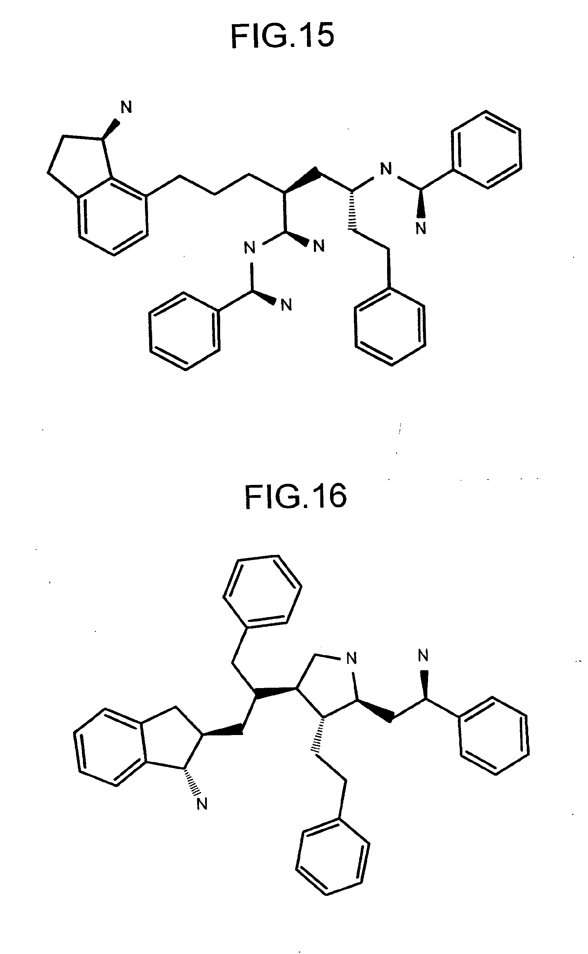

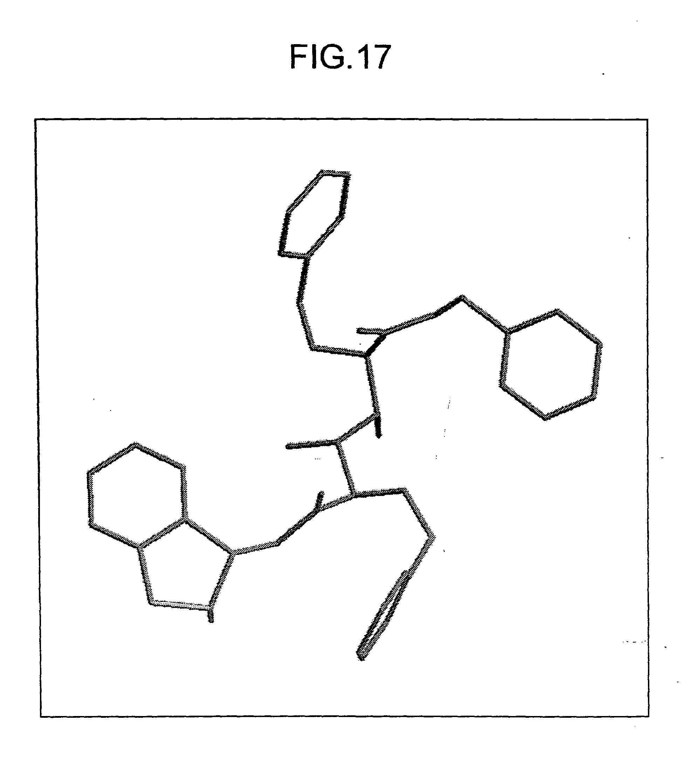

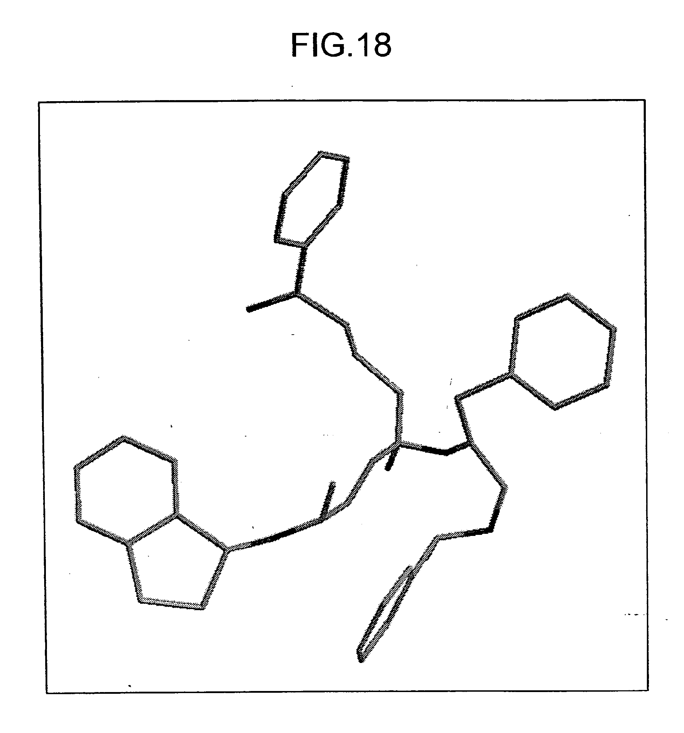

Fig. 13 is a schematic for explaining an example of a known inhibitor structure. Fig. 14 is a schematic for explaining a first example of a candidate ligand structure. Fig. 15 is a schematic for explaining a second example of the candidate ligand structure. Fig. 16 is a schematic for explaining a third example of the candidate ligand structure. Fig. 17 is a schematic of a three-dimensional structure of the known inhibitor shown in Fig. 13. Fig. 18 is a schematic of a three-dimensional structure of the candidate ligand shown in Fig. 14. According to the embodiment, the location quite similar to the measured location of the cyclic structure can be obtained.

The present invention provides a biologically-active-substance candidate structure generation program, a biologically-active-substance candidate structure generation method, and a biologically-active-substance candidate structure generating apparatus capable of efficiently generating an appropriate biologically-active-substance candidate structure.

Although the invention has been described with respect to a specific embodiment for a complete and clear disclosure, the appended claims are not to be thus limited but are to be construed as embodying all modifications that may occur to one skilled in the art which fairly fall within the basic teaching herein set forth.

## Claims

1. An apparatus for generating a biologically-active-substance candidate structure, comprising:

   a first unit for selecting fragments of an arbitrary compound;
   a second unit for locating the fragments selected stably in an active site; and
   a third unit for inputting pseudo monoatoms into the active site in which the arbitrary fragments are stably located, and constructing a molecular scaffold by bonding the fragments together, bonding the fragments with the monoatoms, and bonding the monoatoms together.

2. The apparatus according to claim 1, wherein the first unit includes
   a large fragment selecting unit that selects large fragments having either of a predetermined number of atoms or more and a predetermined volume or more; and
   a small fragment selecting unit that selects, after the large fragments having either of a predetermined number of atoms or more and a predetermined volume or more have been selected, small fragments having either of a number of atoms less than the predetermined number and a volume less than the predetermined volume.

3. The apparatus according to claim 1 or 2, wherein the second unit stably locates the fragments in the active site using a molecular dynamics calculation based on three-dimensional structure information on a protein having the active site.

4. The apparatus according to claim 1, 2 or 3, wherein the third unit locates the pseudo monoatoms in the active site at random, and determines a binding probability of each of the pseudo monoatoms based on a determination of whether at least the atomic distances and bond angles fall within an allowable range.

5. The apparatus according to any preceding claim, further comprising a fourth unit for substituting the pseudo monoatoms for which the molecular scaffold is constructed with heteroatoms.

6. The apparatus according to claim 5, wherein the fourth unit determines the effectiveness of substitution with the heteroatoms based on a fluctuation in an energy of an electrostatic interaction.

7. A method of generating a biologically-active-substance candidate structure, comprising steps of:

   selecting fragments of an arbitrary compound;
   locating the fragments selected stably in an active site; and
   inputting pseudo monoatoms into the active site in which the arbitrary fragments are stably located and constructing a molecular scaffold by bonding the fragments together, bonding the fragments with the monoatoms, and bonding the monoatoms together.

8. The method according to claim 7, wherein the selecting step includes
   selecting large fragments having either of a predetermined number of atoms or more and a predetermined volume or more; and
   selecting, after selecting the large fragments having either of a predetermined number of atoms or more and a predetermined volume or more, small fragments having either of a number of atoms less than the predetermined number and a volume less than the predetermined volume.

9. The method according to claim 7 or 8, wherein the locating step includes locating the fragments stably in the active site using a molecular dynamics calculation based on three-dimensional structure information on a protein having the active site.

**10.** The method according to claim 7, 8 or 9, wherein the inputting and constructing step includes locating the pseudo monoatoms in the active site at random and determining a binding probability of each of the pseudo monoatoms based on a determination of whether at least the atomic distances and bond angles fall within an allowable range.

**11.** The method according to any of claims 7 to 10, further comprising a step of substituting the pseudo monoatoms for which the molecular scaffold is constructed at the third step with heteroatoms.

**12.** The method according to claim 11', wherein the substituting includes determining the effectiveness of substitution with the heteroatoms based on a fluctuation in an energy of an electrostatic interaction.

**13.** A computer readable recording medium that stores a computer program for generating a biologically-active-substance candidate structure, the computer program, when executed by a computer, causing the computer to perform the method of any of claims 7 to 12.

**14.** A method of manufacturing a novel biologically-active-substance comprising the steps of:

> identifying the structure of a biologically-active-substance by carrying out the method of any of claims 7 to 12; and
> synthesizing the substance on the basis of the structure so identified.

**15.** A system for manufacturing a novel biologically-active-substance comprising:

> analysing means for analysing known biologically-active-substances to identify partial structures thereof that appear with high frequency;
> first storage means for storing the partial structures thus identified;
> the apparatus of any of claims 1 to 6, arranged to select partial structures from the first storage means as said fragments;
> second storage means for storing the results of processing by said apparatus; and
> synthesizing means for synthesizing the novel biologically-active-substance on the basis of the results stored in said second storage means.

# FIG.1

INITIAL PACKING

S150

MD CALCULATION
S160

S170

EP 1 562 129 A1

# FIG.2

START

DETERMINE THREE-DIMENSIONAL STRUCTURE OF PROTEIN — S201

DETERMINE ACTIVE SITE OF PROTEIN — S202

SELECT FRAGMENT SET — S203

INPUT FRAGMENT SET INTO ACTIVE SITE — S204

PERFORM MD CALCULATION FOR STABLE LOCATION — S205

INPUT MONOATOMS AND EVALUATE BINDING PROBABILITIES — S206

CONSTRUCT MOLECULAR SCAFFOLD BY BINDING — S207

SELECT SUBSTITUENT HETEROATOM CANDIDATES — S208

SUBSTITUTE MONOATOMS WITH HETEROATOMS — S209

CALCULATE ENERGY — S210

CONFIRM SUBSTITUTION WITH HETEROATOMS — S211

CONFIRM FINAL CANDIDATE STRUCTURE — S212

END

# FIG.3

EP 1 562 129 A1

# FIG.4

FRAGMENT LIBRARY — 101

FRAGMENT SELECTING UNIT — 401

MD CALCULATING UNIT — 402

MONOATOM INPUTTING AND BINDING PROBABILITY EVALUATING UNIT — 403

MOLECULAR SCAFFOLD CONSTRUCTING UNIT — 404

HETEROATOM SUBSTITUTING UNIT — 405

THREE-DIMENSIONAL STRUCTURE INFORMATION — 410

ACTIVE SITE INFORMATION — 420

EP 1 562 129 A1

# FIG.5

```
        ┌──────────────┐
        │    START     │
        └──────┬───────┘
               │
    ┌──────────▼───────────┐
    │ CALCULATE NUMBER     │  S501
    │ OF ACCOMMODATED      │
    │ ATOMS                │
    └──────────┬───────────┘
               │
    ┌──────────▼───────────┐
    │ SELECT LARGE         │  S502
    │ FRAGMENTS            │
    │ RANDOMLY             │
    └──────────┬───────────┘
               │
          ◇ S503
     No  ╱ DOES        ╲
    ◄───╱ ACCOMMODATION ╲
        ╲ CAPACITY REACH╱
         ╲   30%?      ╱
          ◇
          │ Yes
    ┌──────────▼───────────┐
    │ SELECT SMALL         │  S504
    │ FRAGMENTS            │
    │ RANDOMLY             │
    └──────────┬───────────┘
               │
          ◇ S505
     No  ╱ DOES        ╲
    ◄───╱ ACCOMMODATION ╲
        ╲ CAPACITY REACH╱
         ╲   50%?      ╱
          ◇
          │ Yes
    ┌──────────▼───────────┐
    │   STORE RESULT       │  S506
    └──────────┬───────────┘
               │
          ◇ S507
        ╱ HAS          ╲
       ╱ PREDETERMINED  ╲  No
       ╲ SET BEEN       ╱────►
        ╲ SELECTED?    ╱
          ◇
          │ Yes
        ┌──────▼───────┐
        │    END       │
        └──────────────┘
```

# FIG.6

START

EXTRACT FRAGMENT
SET RANDOMLY — S601

LOCATE FRAGMENTS
IN ACTIVE SITE
PROPERLY — S602

EXECUTE MD
CALCULATION — S603

IS INTERACTION
BETWEEN PROTEIN AND
FRAGMENTS HIGH? — S604

No

Yes

STORE RESULT — S605

HAVE
ALL FRAGMENT SETS
BEEN COMPLETED WITH
PROCESSING? — S606

No

Yes

END

# FIG.7

```
                    START

         ┌──────────────────────────┐
         │                          │
                                        S701
              ARE PAIRED
           FRAGMENTS WITHIN          No
           STEREOCHEMICALLY  ─────────────┐
           ALLOWABLE RANGE               │
                 ?                        │
                                          │         S703
              Yes        S702         No  │
                                   ◄──────┤    ARE FRAGMENTS
         BOND FRAGMENTS                   │    TOO CLOSE?
           TOGETHER                       │
                                          │       Yes
                                                    │
                 HAVE        S704                   │
              ALL PAIRED                            │
         No   FRAGMENTS BEEN                        │
              BONDED                                │
              TOGETHER                              │
                 ?                                  │
                                                    │
                     Yes                            │
                                                    │
              INPUT            S705                  │
             MONOATOM                               │
                                                    │
                ARE BOND       S706                 │
              DISTANCE AND                No        │
           BOND ANGLE WITHIN  ──────────────►       │
              APPROPRIATE                           │
               RANGES?                              │
                                                    │
                  Yes                               │
                                                    │
              PERFORM        S707                   │
              BONDING                               │
                                           S708     │
        Yes    ARE NEW BONDS                        │
               GENERATED?                           │
                                                    │
                     No  ◄──────────────────────────┘

                    END
```

# FIG.8

| CONDITIONS | SPECIFIC EXAMPLES |
|---|---|
| (1)      DISTANCE TO EVERY PROTEIN ATOM IS EQUAL TO OR LARGER THAN LIMIT, AND DISTANCE TO HYDROGEN-BONDING ATOM DIFFERS FROM DISTANCE TO NON-HYDROGEN-BONDING ATOM. | DISTANCE TO HYDROGEN-BONDING ATOM 0.26 nanometer DISTANCE TO NON-HYDROGEN-BONDING ATOM 0.33 nanometer |
| (2)      DISTANCE TO ANY ONE OF ALREADY LOCATED ATOMS IS WITHIN STANDARD BOND DISTANCE RANGE | 0.12 nm to 0.16 nm |
| (3)      NUMBER OF ATOMS SATISFYING CONDITION (2) IS FOUR OR LESS. | |
| (4)      BOND ANGLE TO BE GENERATED IS WITHIN PREDETERMINED RANGE. | 100°   OR MORE 130°   OR LESS |
| (5)      AMONG ALREADY LOCATED ATOMS, DISTANCES TO ATOMS OTHER THAN BONDED ATOMS AND ATOMS CONSTITUTING BOND ANGLES AND DIHEDRAL ANGLES ARE EQUAL TO OR LARGER THAN LIMIT | 0.33 nm |

EP 1 562 129 A1

# FIG.9

LOCATION OF TRIAL

# FIG.10

```
            ┌─────────────┐
            │    START    │
            └─────────────┘
                   │
                   ▼
        ┌──────────────────────┐
        │ SUBSTITUTE ATOM WITH │── S1001
        │ HETEROATOM SUITABLE  │
        │   FOR INTERACTION    │
        └──────────────────────┘
                   │
                   ▼
              ╱─────────╲  S1002
             ╱    ARE    ╲        Yes
  ◄─────────╱ N, O, S, P, F, Cl, OR Br ╲──────────┐
            ╲  SUBSTITUENT  ╱                      │
             ╲ CANDIDATES? ╱                       ▼
              ╲──────────╱               ┌──────────────────────┐
                   │ No                  │ SUBSTITUTE ATOM WITH │── S1003
                   ▼                     │ N, O, S, P, F, Cl, OR Br │
        ┌──────────────────────┐        └──────────────────────┘
        │ SUBSTITUTE ATOM WITH │── S1004           │
        │     CARBON ATOM      │                   │
        └──────────────────────┘◄──────────────────┘
                   │
                   ▼
        ┌──────────────────────┐
        │   CALCULATE ENERGY   │── S1005
        └──────────────────────┘
                   │
                   ▼
              ╱─────────╲  S1006
             ╱           ╲       No
            ╱ IS ENERGY REDUCED? ╲──────────┐
             ╲           ╱                   │
              ╲─────────╱                    │
                   │ Yes                     │
                   ▼                         │
        ┌──────────────────────┐            │
        │ CONFIRM SUBSTITUTION │── S1007     │
        │   WITH HETEROATOM    │            │
        └──────────────────────┘            │
                   │◄───────────────────────┘
                   ▼
              ╱─────────╲  S1008
             ╱    ARE    ╲
   No      ╱ ALL CANDIDATES ╲
  ◄───────╱ COMPLETED WITH  ╲
           ╲  PROCESSING?   ╱
             ╲─────────────╱
                   │ Yes
                   ▼
            ┌─────────────┐
            │     END     │
            └─────────────┘
```

FIG.11A

EP 1 562 129 A1

# FIG.11B

x-z plane

FIG.12A

x-y plane

# FIG.12B

x-z plane

# FIG.13

# FIG.14

# FIG.15

# FIG.16

# FIG.17

# FIG.18

**European Patent Office**

## PARTIAL EUROPEAN SEARCH REPORT

which under Rule 45 of the European Patent Convention shall be considered, for the purposes of subsequent proceedings, as the European search report

Application Number

EP 04 25 5094

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | BOHM H-J: "THE COMPUTER PROGRAM LUDI: A NEW METHOD FOR THE DE NOVO DESIGN OF ENZYME INHIBITORS" JOURNAL OF COMPUTER-AIDED MOLECULAR DESIGN, ESCOM SCIENCE PUBLISHERS BV, vol. 6, no. 1, February 1992 (1992-02), pages 61-78, XP000560808 ISSN: 0920-654X * the whole document * ----- | 1-15 | G06F17/50 C07K1/00 G06F19/00 |
| X | BOEHM H-J: "LUDI: RULE-BASED AUTOMATIC DESIGN OF NEW SUBSTITUENTS FOR ENZYME INHIBITOR LEADS" JOURNAL OF COMPUTER-AIDED MOLECULAR DESIGN, ESCOM SCIENCE PUBLISHERS BV, vol. 6, no. 6, December 1992 (1992-12), pages 593-606, XP001008405 ISSN: 0920-654X * the whole document * ----- | 1-15 | |
| | -/-- | | **TECHNICAL FIELDS SEARCHED (Int.Cl.7)** G06F C07K |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 15 June 2005 | Wimmer, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

**European Patent Office**  **PARTIAL EUROPEAN SEARCH REPORT**  Application Number

EP 04 25 5094

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | LUO Z ET AL:  "RASSE: a new method for structure-based drug design." JOURNAL OF CHEMICAL INFORMATION AND COMPUTER SCIENCES. 1996 NOV-DEC, vol. 36, no. 6, November 1996 (1996-11), pages 1187-1194, XP002331826 ISSN: 0095-2338 * the whole document * ----- | 1-15 | |
| X | FRENKEL D ET AL:  "PRO-LIGAND: AN APPROACH TO DE NOVO MOLECULAR DESIGN. 4. APPLICATIONTO THE DESIGN OF PEPTIDES" JOURNAL OF COMPUTER-AIDED MOLECULAR DESIGN, ESCOM SCIENCE PUBLISHERS BV, vol. 9, no. 3, 1 June 1995 (1995-06-01), pages 213-225, XP000674802 ISSN: 0920-654X * the whole document * ----- | 1-15 | |
| X | MOON J B ET AL:  "COMPUTER DESIGN OF BIOACTIVE MOLECULES: A METHOD FOR RECEPTOR- BASED DE NOVO LIGAND DESIGN" PROTEINS: STRUCTURE, FUNCTION AND GENETICS, ALAN R. LISS, US, vol. 11, January 1991 (1991-01), pages 314-328, XP000560842 ISSN: 0887-3585 * the whole document * ----- | 1-15 | TECHNICAL FIELDS SEARCHED (Int.Cl.7) |
| X | EP 0 818 744 A (PROTEUS MOLECULAR DESIGN LIMITED) 14 January 1998 (1998-01-14) * the whole document * ----- | 1-15 | |
| X | EP 1 010 681 A (ITAI, AKIKO) 21 June 2000 (2000-06-21) * the whole document * ----- | 1-15 | |
| | -/-- | | |

EPO FORM 1503 03.82 (P04C10)

# EP 1 562 129 A1

**PARTIAL EUROPEAN SEARCH REPORT**

Application Number

EP 04 25 5094

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | HONMA T: "Recent advances in de novo design strategy for practical lead identification" MEDICINAL RESEARCH REVIEWS, NEW YORK, NY, US, vol. 23, no. 5, September 2003 (2003-09), pages 606-632, XP002326365 ISSN: 0198-6325 * the whole document * ----- | 1-15 | |
| T | DOUGUET DOMINIQUE ET AL: "LEA3D: a computer-aided ligand design for structure-based drug design." JOURNAL OF MEDICINAL CHEMISTRY. 7 APR 2005, vol. 48, no. 7, 7 April 2005 (2005-04-07), pages 2457-2468, XP002331827 ISSN: 0022-2623 * the whole document * ----- | 1-15 | |

TECHNICAL FIELDS SEARCHED (Int.Cl.7)

EPO FORM 1503 03.82 (P04C10)

**European Patent Office**

**INCOMPLETE SEARCH**
**SHEET C**

Application Number

EP 04 25 5094

Claim(s) searched incompletely:
        1-15

Reason for the limitation of the search:

The term "pseudo monoatom" as used in the claims is not clear to the skilled person, and a specific meaning of the term could not be derived from the description. The search was therefore carried out with respect to general ligand-design methods, in particular those using fragment-fitting.

**ANNEX TO THE EUROPEAN SEARCH REPORT
ON EUROPEAN PATENT APPLICATION NO.**

EP 04 25 5094

This annex lists the patent family members relating to the patent documents cited in the above-mentioned European search report.
The members are as contained in the European Patent Office EDP file on
The European Patent Office is in no way liable for these particulars which are merely given for the purpose of information.

15-06-2005

| Patent document cited in search report | | | Publication date | Patent family member(s) | | Publication date |
|---|---|---|---|---|---|---|
| EP 0818744 | A | | 14-01-1998 | EP 0818744 | A2 | 14-01-1998 |
| EP 1010681 | A | | 21-06-2000 | AU 1152897 | A | 28-07-1997 |
| | | | | EP 1010681 | A1 | 21-06-2000 |
| | | | | CN 1209111 | A | 24-02-1999 |
| | | | | WO 9724301 | A1 | 10-07-1997 |
| | | | | US 2002062155 | A1 | 23-05-2002 |

EPO FORM P0459